# EUROPEAN PATENT SPECIFICATION

(11) **EP 4 305 397 B1**
(45) Date of publication and mention of the grant of the patent: **22.04.2026**
(21) Application number: 22770064.8
(22) Date of filing: 11.03.2022
(51) Int. Cl.: G01N 1/24, C12Q 1/04, G01N 33/569, G01N 1/40, G01N 1/22

(54) **METHOD FOR EXTRACTION OF MYCOTOXINS FROM A SAMPLE**
VERFAHREN ZUR EXTRAKTION VON MYKOTOXINEN AUS EINER PROBE
MÉTHODE D'EXTRACTION DE MYCOTOXINES D'UN ÉCHANTILLON

(30) Priority: 13.03.2021 AU 2021900729; 01.08.2021 AU 2021902370
(43) Date of publication of application: 17.01.2024
(73) Proprietor: Goldsworthy, Robert, Sydney, NSW 2000 (AU); Goldsworthy, Susan, Sydney, NSW 2000 (AU)
(72) Inventor: Goldsworthy, Robert, Sydney, NSW 2000 (AU); Goldsworthy, Susan, Sydney, NSW 2000 (AU)
(74) Representative: London IP Ltd
(86) International application number: PCT/AU2022/050204
(87) International publication number: WO 2022/192936

(56) References cited:
- FR-A1- 2 913 502
- KR-A- 20130 014 713
- US-A- 4 772 551
- US-A1- 2005 208 606
- US-A1- 2005 208 606
- US-A1- 2017 336 298
- VOJDANI ARISTO ET AL: "Antibodies against molds and mycotoxins following exposure to toxigenic fungi in a water-damaged building", ARCHIVES OF ENVIRONMENTAL HEALTH, WASHINGTON DC, US, vol. 58, no. 6, 1 June 2004 (2004-06-01), pages 324 - 336, XP009184488, ISSN: 0003-9896
- POTTIER DIDIER ET AL: "Airborne molds and mycotoxins in Serpula lacrymans -damaged homes", ATMOSPHERIC POLLUTION RESEARCH, vol. 5, no. 2, 1 April 2014 (2014-04-01), pages 325 - 334, XP093226731, ISSN: 1309-1042, DOI: 10.5094/APR.2014.038
- BRASEL T L ET AL: "DETECTION OF AIRBORNE STACHYBOTRYS CHARTARUM MACROCYCLIC TRICHOTHECENE MYCOTOXINS ON PARTICULATES SMALLER THAN CONIDIA", APPLIED AND ENVIRONMENTAL MICROBIOLOGY, AMERICAN SOCIETY FOR MICROBIOLOGY, US, vol. 71, no. 1, 1 January 2005 (2005-01-01), pages 114 - 122, XP002388359, ISSN: 0099-2240, DOI: 10.1128/AEM.71.1.114-122.2005
- JOHANNING E ET AL: "AIRBORNE MYCOTOXIN SAMPLING AND SCREENING ANALYSIS", PROCEEDINGS: INDOOR AIR 2002, 9TH INTERNATIONAL CONFERENCE ON INDOOR AIR QUALITY AND CLIMATE 2002, 31 December 2002 (2002-12-31), XP055971072
- BRASEL, T.L. ET AL.: "Detection of Airborne Stachybotrys chartarum Macrocyclic Trichothecene Mycotoxins on Particulates Smaller than Conidia", AMERICAN SOCIETY FOR MICROBIOLOGY APPLIED AND ENVIRONMENTAL MICROBIOLOGY, vol. 71, no. 1, 1 January 2005 (2005-01-01), pages 114 - 122, XP002388359, Retrieved from the Internet <URL:https://journals.asm.org/doi/epub/10.1128/AEM.71.1.114-122.2005> DOI: 10.1128/AEM.71.1.114-122.2005
- JOHANNING E, GAREIS M, NIELSEN K, DIETRICH R, MÄRTLBAUER E: "AIRBORNE MYCOTOXIN SAMPLING AND SCREENING ANALYSIS", PROCEEDINGS: INDOOR AIR 2002, 9TH INTERNATIONAL CONFERENCE ON INDOOR AIR QUALITY AND CLIMATE 2002, 31 December 2002 (2002-12-31), XP055971072

## Description

### Background

The invention relates to a method for extraction of mycotoxins from a sample. In particular, the invention relates to a non-gravimetric method for extraction of airborne mycotoxins present in a sample collected from air and quantification of the extracted mycotoxins.

### Background

Fungus such as mould may produce chemical by-products known as mycotoxins. Such mycotoxins may be hazardous to human health when such mycotoxins are present as a result of mould or water damage either currently or historically active within the built environment such as within a person's home.

Accordingly, methods and apparatuses have been developed to sample and test for mould within the built environment. Some methods may use a vacuum to collect mould spore samples from surfaces and then the samples may be tested to identify the mould type such as by crushing the spores to expose the DNA and identify the mould based on the DNA profile.

A problem with such identification of the mould species is that it does not specifically identify or allow quantification of the associated hazardous mycotoxins that may be present or to which a person may be exposed.

To more specifically quantify mycotoxins present in the indoor environment. A method to sample air from the indoor environment and collect mycotoxins from the air has been developed. WO/2021/081578 discloses such a method which includes collection of a mycotoxin sample from an air space of an indoor environment, the method may include conditioning air, such as with an air agitator, within an air space to provide conditioned air representative of air exposed to a person within the indoor environment and sampling the air such as by filtering the conditioned air at a height within the airspace further representative of air exposed to a person within the indoor environment so as to obtain the mycotoxin sample. The collected sample may then be extracted from the sample and analysed.

Air samples are different to sample extraction of a commodity (such as a mechanically captured sample) in that the air sample consists of finer, almost gaseous, particulates that have been captured within a sample collector such as a filter. Air samples may not require grinding, or any further filtration.

Existing methods of processing samples of commodities typically include steps, not appropriate for air samples, such as grinding a measured portion of the commodity in question. Another step, not appropriate for air samples, is the filtering of larger particulate matter from the ground commodity.

The invention disclosed herein seeks to overcome one or more of the above identified problems or at least provide a useful alternative.

US2005/208606 discloses a method of detecting the presence of an airborne mycotoxin in an enclosure. KR20130014713 discloses a method for rapid detecting mycotoxins in fermented tea.

### Summary

In accordance with a first broad aspect there is provided, a method to extract a mycotoxin, or a plurality of mycotoxins, from a sample carrier into a solvent solution according to claim 1.

In an aspect, the water is deionised water.

In another aspect, the step of immersing includes submersing the sample carrier within the solvent solution.

In yet another aspect, the vessel is a test tube.

In yet another aspect, the step of removing the sample carrier from the vessel includes squeeze drying the sample carrier such that excess solvent solution is drained into the vessel.

### Brief Description of the Figures

The invention is described, by way of non-limiting example only, by reference to the accompanying figures, in which;

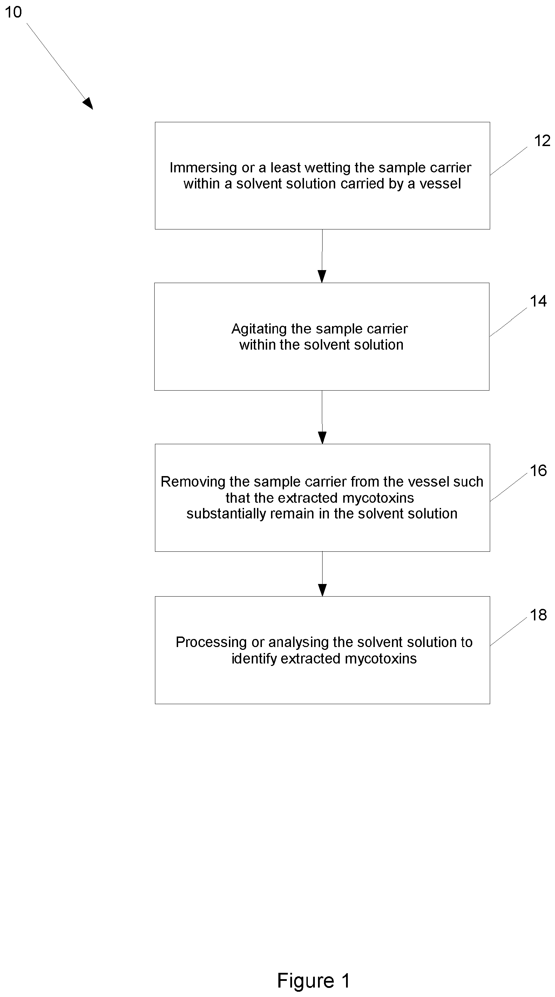

Figure 1 is a flow chart illustrating an example method for extraction of mycotoxins from a sample.

### Detailed Description

Mycotoxins, including but not limited to, Aflatoxins B1, B2, G1, G2, Ochratoxin A, Fumonisin, Vomitoxin, Zearelenone, T-2, HT-2, may be targeted through the below described respiratory size collection methods and then undergo direct analysis via quantitative analysis to determine the airborne concentration of given mycotoxins within the breathable air and reported in ppb (Parts per Billion) relative to the volume of air collected during the sampling period.

Mycotoxins may be collected from air, such as air of the indoor environment, by a variety of techniques. WO/2021/081578 discloses such a method which includes collection of a mycotoxin sample from an air space of an indoor environment using one or more filters. The filters are disclosed as including, for example, a primary filter including a high-density foam, such as polyurethane foam, adapted to capture mycotoxins from the air drawn therethrough and a secondary filter that may be a PVC filter (Polyvinyl chloride). Such filters provide a sample carrier (not shown) in which mycotoxins are captured and carried until extracted. Accordingly, there is a need for a method to extract the mycotoxins from the sample carrier for analysis and quantification.

Referring to Figure 1, there is shown a method 10 for the extraction of mycotoxins from a sample carrier or collector (not shown) for analysis and quantification. Such a mycotoxin is described herein as an initially airborne mycotoxin - which has been captured by the sample carrier or collector. The captured mycotoxins may still be airborne within air pockets or the like within the sample carrier or collector and thereby need to be removed from the air and sample carrier into a solution for processing.

The sample carrier comprises a primary filter formed of polyurethane high-density foam adapted to capture mycotoxins from the air drawn therethrough and a secondary filter formed of polyvinyl chloride (PVC). The filters have been used to collect the mycotoxins samples by drawing air through the primary filter and then the secondary filter. The polyurethane foam and PVC filter are collectively referred to here as "filters" that are used as the sample carrier or collector in which airborne mycotoxins have been collected for extraction. The initially airborne mycotoxins are located within air pockets or within or on the material structures of the filter and need to be extracted or "stripped" from the filter.

The method 10 includes at step 12, immersing the sample carrier within a solvent solution carried by a vessel to dissolve at least some or most of the captured mycotoxins into the solvent solution with permeates the sample carrier, at step 14 agitating the sample carrier within the solvent solution to extract some or most of the captured mycotoxins from the sample carrier into the solvent solution, and a step 16 removing the sample carrier from the vessel such that the extracted mycotoxins substantially remain in the solvent solution. Immersion assists here to ensure the airborne mycotoxins within the carrier medium, in this case the filter, become dissolved within the solvent solution. At step 18, once extracted, the method further includes processing or analysing the solvent solution to identify extracted mycotoxins.

Turning to the method 10 now in more detail, the solvent solution includes about 70% methanol and about 30% water. The water may be deionised water solvent solution.

It has been found that methanol is advantageous in stripping and dissolving Mycotoxins. It is believed that a reason for this is that a Mycotoxin behaves like a lipoid and dissolves most suitability into alcohol, in particular methanol. Furthermore, alcohol, in particular methanol has been found to not degrade the sample carrier, such as the foam filter, which means that, for example, parts of the foam filters are not extracted into and contaminate the solvent solution.

The vessel may be a test tube such as a falcon tube, and, for example, about 10 millilitres of the solvent solution may be placed into the test tubes. The sample carrier, such as the foam filter, may be removed from any packaging or storage cartridge and then a user may, using tweezers or the like, grasp the foam and submerse within the solvent solution. It is noted that the vessel in which the foam and filter are to undergo the extraction process must exceed the circumference of the foam and filter to allow correct ultrasonification.

Agitating the sample carrier within the solvent solution includes manual agitation such as the user massaging the foam several times whilst holding in the solution until the sample carrier substantially saturated, being generally full of solution and remains submersed. The sample carrier, if formed of foam, will take on a more translucent appearance. If there are more than one sample carriers, then the process can be repeated for each of the sample carriers. The vessel may then be placed on a rack. Agitation assists to move and extract the airborne mycotoxins from within the sample carrier, in this case the filter.

In addition to manual agitation, the agitating the sample carrier within the solvent solution includes ultrasonication for a time period, such as, but not limited to, 10 minutes. In other examples, the time period may be in the range of 1 to 20 minutes, 5 to 15 minutes or 9 to 11 minutes. This includes placing and removing the rack from an ultrasonication apparatus (not shown). Ultrasonication should occur on a medium setting, that being between 40kHz and 200kHz at a temperature of 20-24 degrees Celsius for a period, that is preferably, no less than 10 minutes.

The step of removing the sample carrier from the vessel includes squeeze drying the sample carrier such that excess solvent solution is drained into the vessel. For example, a user may squeeze the sample carrier such as the foam filter using tweezers and massage it above the water line of the solution until the solution is substantially removed. The sample carrier may take on its original white appearance when the solution is extracted. The sample carrier may then be removed and discarded. The mycotoxins are now extracted into the solution. It has been found that up to about 80% or greater of captured mycotoxins may be extracted by this method into the solvent solution.

The solution may then be further processed to identify and quantify the extracted mycotoxins. After the extraction process and removal of the filter from the liquid, there may also be a further dilution step prior to processing the sample using ELISA (Enzyme-Linked Immunosorbent Assay). The dilution step may involve 1 part sample and 3 parts deionized water. For example, using a pipettor, 1ml amount of the solution may be transferred into a 7ml tube and 3mls of deionized water may be added to form a processing sample.

The processing sample is now ready for processing by a non-gravimetric enzyme-linked immunosorbent assay technique for the desired mycotoxins and the identified mycotoxins can be reported such as in a cubic meter format, more specifically, a parts per cubic meter exposure within a given time period relative to the volume of air collected during the sampling period. Each space, such as a home or workplace, has different size rooms and each occupant uses a space differently and for longer and shorter times. For example, if 1-hour sample is taken within a room, that is the sample that is analysed (allowing our dilution rates etc). If that sample is taken by a pump at a rate of a round figure of, say, 100/litres per hour, then it is known to multiply the result by 10 to report in a cubic meter format (1,000 litres = 1 cubic meter).

Advantageously, there has been disclosed a method for extraction of mycotoxins from a sample into a solvent solution which provides increased yield and targeting of mycotoxins over other methods such as the use of separation columns both specific and non-specific commonly used in food and feed extraction methods. In particular, the method allows for extraction of airborne mycotoxins present in a sample collected from air and analysis and quantitation of such collected airborne mycotoxins. The method enables the results to be reported as a parts per cubic meter exposure within a given time period relative to the volume of air collected during the sampling period which allows the human exposure to mycotoxins to be determined and subsequent test results to follow a repeatable process for on-going comparison.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

## Claims

1. A non-gravimetric method (10) to extract one or more airborne mycotoxins from a sample carrier in which the one or more airborne mycotoxins have been collected by the sample carrier from air, the method including:
Immersing (12) the sample carrier within a solvent solution carried by a vessel, the sample carrier including a primary filter formed of polyurethane high-density foam and a secondary filter formed of polyvinyl chloride, the solvent solution including about 70% methanol and about 30% water to separate the one or more airborne mycotoxins from the primary filter and the secondary filter;
Manually agitating (14) at least the primary filter whilst holding it in the solvent solution until the sample carrier is saturated;
Further agitating the primary filter and secondary filter within the solvent solution using ultrasonic agitation to extract the one or more airborne mycotoxins from the primary filter and the secondary filter into the solvent solution;
Removing (16) the primary filter and secondary filter from the vessel whilst at least partially squeeze drying the primary filter and the secondary filter such that excess solvent solution is drained into the vessel; and
Processing (18) the solvent solution to identify one or more extracted airborne mycotoxins using an enzyme-linked immunosorbent assay technique.

2. The method according to claim 1, wherein the water is deionised water.

3. The method according to claim 1, wherein the vessel is a test tube.

4. The method according to claim 1, wherein the step of removing the sample carrier from the vessel includes squeeze drying the sample carrier such that excess solvent solution is drained into the vessel.

5. The method according to claim 1, wherein the step of manually agitating at least the primary filter includes a user massaging it several times whilst holding it in the solvent solution until the sample carrier substantially saturated.

## Patentansprüche

1. Nicht-gravimetrisches Verfahren (10) zum Extrahieren eines oder mehrerer luftgetragener Mykotoxine aus einem Probenträger, in dem das eine oder die mehreren luftgetragenen Mykotoxine durch den Probenträger aus der Luft eingesammelt wurden, das Verfahren einschließend:
Eintauchen (12) des Probenträgers in eine Lösungsmittellösung, die in einem Behälter enthalten ist, wobei der Probenträger einen primären Filter, der aus Polyurethan-Schaum mit hoher Dichte geformt ist, und einen sekundären Filter, der aus Polyvinylchlorid geformt ist, einschließt und die Lösungsmittellösung etwa 70% Methanol und etwa 30% Wasser einschließt, um das eine oder die mehreren luftgetragenen Mykotoxine aus dem primären Filter und dem sekundären Filter abzuscheiden;
Manuelles Hin- und Herbewegen (14) mindestens des primären Filters, während er in der Lösungsmittellösung gehalten wird bis der Probenträger gesättigt ist;
weiteres Hin- und Herbewegen des primären Filters und des sekundären Filters innerhalb der Lösungsmittellösung unter Verwendung einer Ultraschall-Agitation, um das eine oder die mehreren luftgetragenen Mykotoxine aus dem primären Filter und dem sekundären Filter in die Lösungsmittellösung zu extrahieren;
Entnehmen (16) des primären Filters und des sekundären Filters aus dem Behälter, während der primäre Filter und der sekundäre Filter mindestens teilweise ausgedrückt werden, um zu trocknen, sodass überschüssige Lösungsmittellösung in den Behälter zurücktropft; und
Verarbeiten (18) der Lösungsmittellösung, um ein oder mehrere luftgetragene Mykotoxine unter Verwendung eines enzymgekoppelten Immunadsorptionstests zu identifizieren.

2. Verfahren nach Anspruch 1, wobei das Wasser entionisiertes Wasser ist.

3. Verfahren nach Anspruch 1, wobei der Behälter ein Reagenzglas ist.

4. Verfahren nach Anspruch 1, wobei der Schritt des Entfernens des Probenträgers aus dem Behälter das Ausdrücken des Probenträgers zum Trocknen einschließt, um die überschüssige Lösungsmittellösung in den Behälter zurücktropfen zu lassen.

5. Verfahren nach Anspruch 1, wobei der Schritt zum manuellen Hin- und Herbewegen mindestens des ersten Filters einschließt, dass er von einem Benutzer mehrmals zusammengepresst wird, während er in die Lösungsmittellösung eingetaucht ist, bis der Probenträger im Wesentlichen gesättigt ist.

## Revendications

1. Procédé non gravimétrique (10) pour extraire une ou plusieurs mycotoxines en suspension dans l'air d'un porte-échantillon par lequel la ou les mycotoxines en suspension dans l'air ont été prélevées par le porte-échantillon de l'air, le procédé comportant :
l'immersion (12) du porte-échantillon à l'intérieur d'une solution de solvant transportée par un récipient, le porte-échantillon comportant un filtre primaire formé de mousse de polyuréthane haute densité et un filtre secondaire formé de chlorure de polyvinyle, la solution de solvant comportant environ 70 % de méthanol et environ 30 % d'eau pour séparer la ou les mycotoxines en suspension dans l'air du filtre primaire et du filtre secondaire ;
l'agitation manuelle (14) d'au moins le filtre primaire tout en le maintenant dans la solution de solvant jusqu'à saturation du porte-échantillon ;
l'agitation supplémentaire du filtre primaire et du filtre secondaire à l'intérieur de la solution de solvant au moyen d'une agitation par ultrasons pour extraire la ou les mycotoxines en suspension dans l'air du filtre primaire et du filtre secondaire dans la solution de solvant ;
le retrait (16) du filtre primaire et du filtre secondaire du récipient tout en effectuant au moins partiellement le séchage par sous pression mécanique du filtre primaire et du filtre secondaire de sorte que la solution de solvant excédentaire soit évacuée dans le récipient ; et
le traitement (18) de la solution de solvant pour identifier une ou plusieurs mycotoxines extraites en suspension dans l'air au moyen d'une technique de dosage immunoenzymatique.

2. Procédé selon la revendication 1, dans lequel l'eau est de l'eau désionisée.

3. Procédé selon la revendication 1, dans lequel le récipient est un tube à essai.

4. Procédé selon la revendication 1, dans lequel l'étape consistant à retirer le porte-échantillon du récipient comporte le séchage par sous pression mécanique du porte-échantillon de sorte que la solution de solvant en excès soit évacuée dans le récipient.

5. Procédé selon la revendication 1, dans lequel l'étape consistant à agiter manuellement au moins le filtre primaire comporte un massage par un utilisateur de celui-ci plusieurs fois tout en le maintenant dans la solution de solvant jusqu'à ce que le porte-échantillon soit pratiquement saturé.
